# EUROPEAN PATENT APPLICATION

(11) **EP 2 664 914 A1**
(43) Date of publication of application: **20.11.2013**
(21) Application number: 12169019.2
(22) Date of filing: 23.05.2012
(51) Int. Cl.: G01N 27/74

(54) **Magnetically assisted processing of a medium**

(30) Priority: 16.05.2012 US 201261647551 P
(71) Applicant: Koninklijke Philips N.V., 5656 AE Eindhoven (NL)
(72) Inventor: The designation of the inventor has not yet been filed
(74) Representative: Jungen, Carmen

(57) **Abstract**

The invention relates to a processing device (100) and a method for processing a medium in a processing chamber (121). The processing comprises the addition of magnetic particles (M) to the medium and the mixing of the medium by manipulating said magnetic particles with a time-variable magnetic field (B), particularly a partially oscillating or rotating field. The magnetic field (B) may be generated with a multipole magnetic field generator (110) comprising four subunits (111A, 111B), each having a core (113A, 113B) with a surrounding coil (112A, 112B) and with a top surface (114A, 114B), wherein all top surfaces of said subunits are preferably arranged in the same plane and wherein all cores are substantially parallel to each other.

## Description

### FIELD OF THE INVENTION

The invention relates to a processing device and a method that can be used to magnetically process a medium in a processing chamber.

### BACKGROUND OF THE INVENTION

The WO 2010/044006A2 discloses a biosensor system comprising a quadrupole magnetic assembly with four subunits. Each subunit comprises a core, a top surface, and a coil. The magnetic assembly is used to generate a magnetic field at a sensor surface by which magnetic particles can be attracted to the sensor surface or removed from this surface.

### SUMMARY OF THE INVENTION

It is an object of the invention to provide means that allow for a more versatile processing of a medium in a processing chamber.

This object is achieved by a method according to claim 1 and a processing device according to claim 2. Preferred embodiments are disclosed in the dependent claims.

According to a first aspect, the invention relates to a method for processing a medium comprising target particles, for example a biological sample fluid comprising nucleic acids as target particles. The method comprises the following steps, which may be executed in the listed or any other appropriate order:
a) Providing a cartridge that has a processing chamber which can be filled with the medium to be processed. The cartridge may typically be an exchangeable (disposable) component made e.g. of glass or plastic by injection molding.
b) Providing a magnetic field generator with at least four subunits, each subunit having a core with a top surface and a coil surrounding said core, wherein the top surfaces of all subunits are arranged adjacent to the aforementioned cartridge (and thus adjacent to the processing chamber). For purposes of reference, this magnetic field generator will in the following be called "multipole magnetic field generator" (wherein the multipole has at least four poles).
c) Filling the processing chamber with the medium comprising the target particles. Moreover, magnetic particles (M) are added to the medium before, during, and/or after the filling step. In this context, the term "magnetic particles" shall comprise both permanently magnetic particles as well as magnetizable particles, for example superparamagnetic beads. The size of the magnetic particles typically ranges between 3 nm and 50 µm.
d) Controlling the multipole magnetic field generator such that a time-variable magnetic field is generated that manipulates the magnetic particles and thus mixes the medium in the processing chamber. Most preferably, the at least four subunits of the multipole magnetic field generator are individually controlled in this step, i.e. supplied with individual drive currents.
e) Binding target particles of the medium to the magnetic particles.
f) Controlling the multipole magnetic field generator such that the magnetic particles are attracted to a surface of the processing chamber and removing the remaining medium from the processing chamber.

An important component that is used in the above method is the "multipole magnetic field generator" with which a magnetic field can be generated that is particularly suited for manipulating a medium like a biological sample fluid. The top surfaces of all subunits of this magnet are preferably arranged in the same plane, and the cores are preferably substantially parallel to each other.

The aforementioned arrangement "in the same plane" shall by definition be measured on a scale that is related to the size of the whole apparatus. More particularly, the fourth top surface shall be considered as being arranged in a plane that comprises or at least intersects the first three top surfaces (such a plane always exists) if its distance from this plane is less than about 10 %, preferably less than about 5 %, most preferably less than about 1 % of the largest distance between two top surfaces.

Similarly, two cores are considered to be "substantially parallel" to each other if the axes of extension of the cores are oriented at a relative angle of less than about 20°, preferably less than about 10°.

In general, the four subunits of the multipole magnetic field generator may differ in their dimensions and/or design. Preferably, all four subunits are however substantially identical to each other. Moreover, they are preferably disposed in an arrangement with rotational symmetry, for example at the corners of a square.

There may be more than four subunits with the features described above (i.e. with a core, a top surface, a coil surrounding the core, and optional further features), wherein the total number of such subunits (or coils) will in general be even.

According to a second aspect, the invention relates to a processing device for processing a medium comprising target particles. The processing device may for example be a biosensor device in which a biological sample fluid can optionally be subjected to certain processing steps and in which properties of the fluid can be measured. The processing device comprises the following components:
- A cartridge with a processing chamber in which the medium to be processed can be provided.
- A "multipole magnetic field generator" that comprises at least four magnetic subunits each having a core with a top surface and a surrounding coil, wherein the top surfaces are arranged adjacent to the cartridge.
- A control unit by which individual drive currents can be supplied to the subunits of the multipole magnetic field generator, comprising drive currents by which
   a) magnetic particles are manipulated to mix a medium in the processing chamber;
   b) magnetic particles are attracted to a surface of the processing chamber.

The processing device and the method are based on the same concept that they allow for the mixing of a medium with the help of a magnetic field that manipulates certain entities in the medium, for example magnetic particles. Additionally, the magnetic field generator has a favorable design that allows its arrangement adjacent to a planar side of a cartridge comprising the medium.

Due to their relationship, explanations provided for the processing device or the method are analogously valid for the other component, too. Moreover, preferred embodiments of the invention will be described in the following that are mutatis mutandis applicable to the processing device and the method.

In a preferred embodiment of the invention, the at least four subunits of the multipole magnetic field generator are arranged below the processing chamber (wherein the term "below" shall not refer to a specific orientation with respect to gravity but shall express that the at least four subunits on the one hand side and the processing chamber on the other hand side are arranged at different sides of some given plane). Preferably, the subunits also arranged below the cartridge (which contains the processing chamber). The cartridge can then favorably be placed on top of the multipole magnetic field generator.

According to another preferred arrangement, the at least four subunits of the multipole magnetic field generator are arranged to surround the processing chamber. In particular, the top surfaces of the subunits may be arranged such that the processing chamber lies (at least partially) between them. The at least four subunits may for example protrude into the cartridge, thus surrounding the processing chamber.

In another embodiment of the invention, an additional magnetic field generator may be provided that is arranged opposite to the multipole magnetic field generator with respect to the processing chamber (i.e. the processing chamber is at least partially disposed between the multipole magnetic field generator and the additional magnetic field generator). The additional magnetic field generator may for example be a permanent magnet or an electromagnet. Moreover, it is preferred that the additional magnetic field generator can be controlled independently of the multipole magnetic field generator.

The magnetic particles that are added to the medium to be processed preferably form magneto-rheological structures while they are manipulated to mix the medium. Such structures may for example comprise a complicated network of chains of magnetic particles.

The volume of the processing chamber that is filled with the medium to be processed is typically smaller than about 2000 µl, preferably smaller than about 500 µl and could be down to 10 µl, possibly even 1 µl. Mixing of such small fluid volumes is a problem as conventional approaches based on the generation of turbulences typically fail. Accordingly, it is a great advantage that the present invention allows for a magnetically induced mixing of such small volumes.

The magnetic particles that are added to the medium in the first step of the method are preferably distributed (uniformly or non-uniformly) over the whole processing chamber during the step of mixing. Accordingly, the magnetic coupling and mixing takes substantially place within the whole processing chamber.

The magnetic field that is generated by the magnetic field generator(s) and/or the time-variable magnetic field that is applied during the mixing step of the method preferably comprises at least one oscillating (vector) component. Moreover, said magnetic field may at least partially be rotating (i.e. it may consist of a vector rotating in a given, stationary plane and a vector perpendicular to said plane). A magnetic field with an oscillating component and/or a partially rotating magnetic field can induce corresponding oscillating and/or rotating movements of magnetic particles that have turned out to be efficient mixing operations.

The frequency of the aforementioned oscillations or rotations preferably ranges between about 0.1 Hz and about 100 Hz.

According to another embodiment of the invention, all or some of the magnetic particles that are added to the medium comprise binding sites which can specifically bind to certain target components of the medium. The magnetic particles may for example be coated with antigens that specifically bind to antibodies in a biological sample medium. After adding such magnetic particles to the medium, binding between magnetic particles and target components (if present) will take place, which is an effect of the magnetic particles additional to the mixing of the medium.

In still another embodiment of the invention, all or some of the magnetic particles that are added to the medium are capable of electrostatically binding charged and/or polarized particles (e.g. molecules), particularly fragments of nucleic acids like DNA or RNA or other nucleic acids.

The strength of the aforementioned electrostatic binding will usually depend on the magnitude of the charge of the bound molecules. For fragments of nucleic acids, the magnitude of their charge is typically related to the size of the fragments. The electrostatic binding of fragments of nucleic acids to magnetic particles can therefore be used to selectively separate small fragments (that do not sufficiently bind to the magnetic particles) from long ones (that do sufficiently bind).

In a processing step of the method, the magnetic particles are attracted to a surface of the processing chamber. Such an attraction to a surface can for instance be used to prepare the bulk medium in the processing chamber for processing steps without the magnetic particles. Additionally or alternatively, specific processing steps may be executed with the magnetic particles and/or components attached thereto at the surface.

In another embodiment of the invention, a new medium is introduced into the processing chamber during the attraction of the magnetic particles to a surface. Thus an isolated exchange of the media is possible while the magnetic particles remain within the processing chamber. If the magnetic particles bind target components of a first medium, these target components can be retained in the processing chamber, too, when a new medium is introduced. The magnetic particles may hence for instance be used to retain long fragments of nucleic acids in the processing chamber while short fragments are removed together with the bulk medium.

According to a further development of the aforementioned embodiment, target components that are bound to magnetic particles (particularly target components that have been bound to magnetic particles during the binding step of the method) are released into the new medium. This means that these target components dissociate from the magnetic particles after the new medium has been introduced into the processing chamber. The whole procedure therefore corresponds to a specific transfer of target components from one medium to another, which may be used to purify and/or up-concentrate biological samples.

The steps of attracting magnetic particles to a surface of the processing chamber, exchanging the medium in said chamber, and/or magnetically mixing the medium in the chamber can be repeated several times in any appropriate order as required by a particular assay. Thus it is for example possible to realize an efficient DNA purification procedure.

In general, the target particles in the medium that is processed may preferably comprise nucleic acids like DNA or RNA, proteins, polypeptides, lipids, carbohydrates, metabolites, hormones, drugs, pharmaceutical materials, cell fragments, cells, tissue elements or a mixture of some of the aforementioned components.

It was already mentioned that the processing of the medium may particularly be or comprise a detection procedure. According to a related embodiment, the processing device may comprise a light detector for detecting light that was totally internally reflected at the planar wall of the processing chamber. It is then possible to execute measurements with the favorable method of frustrated total internal reflection (FTIR) at the surface of said wall.

Details of this method may be found in the US 2011/0221427 A1 or the WO 2008/072156 A2, which are incorporated into the present application by reference.

The processing device and/or the magnetic field generator(s) may preferably comprise a control unit for selectively applying drive currents to the coils of the subunits (and/or to the additional magnetic field generator, if present). Thus it is possible to control each subunit individually, which allows for a versatile generation of magnetic fields within the adjacent space or processing chamber.

The aforementioned drive currents may for example have a sinusoidal time course, wherein the currents of different subunits may have the same frequencies but different phases. In general, the frequencies and/or amplitudes of the currents may be different for different subunits. Other possible time courses of drive currents comprise square, triangle, sawtooth or irregular waveforms.

According to a third aspect, the invention relates to a processing device for processing a medium comprising target particles, said processing device comprising the following components:
- a cartridge with a processing chamber in which the medium can be provided;
- a multipole magnetic field generator with at least four subunits, each subunit having a core with a top surface and a coil surrounding said core, wherein the top surfaces of all subunits are arranged adjacent to the cartridge;
- an additional magnetic field generator that is arranged opposite to the multipole magnetic field generator with respect to the processing chamber;
- a control unit by which individual drive currents can be supplied to the subunits of the multipole magnetic field generator and/or to the additional magnetic field generator.

The described processing device allows for a versatile manipulation of magnetic particles in the processing chamber because the latter is enclosed between a multipole magnetic field generator and an additional magnetic field generator which can individually be controlled. Hence it is for example possible to mix small volumes of a medium in the processing chamber by manipulating the magnetic particles appropriately.

The processing device according to the third aspect can optionally have one or more of the features described above with respect to the processing device and/or the method according to the first and second aspect of the invention, respectively.

The invention further relates to the use of the processing device according to the first and/or the third aspect of the invention for mixing a medium by actuating magnetic particles. As described above, this mixing can particularly comprise the generation of a time-variable magnetic field that manipulates the magnetic particles to form magneto-rheological structures.

Moreover, the invention relates to the use of the processing device according to the first and/or the third aspect of the invention for the separation of target components from a medium, particularly for the separation of DNA fragments (or other nucleic acid substances) of specific lengths from other DNA fragments (or nucleic acids substances). Thus it is for example possible to realize an efficient purification and removal of short oligo- and DNA fragments, e.g. adapters from a DNA library.

The invention further relates to the use of the processing devices described above for molecular diagnostics, biological sample analysis, nucleic acid processing, particularly nucleic acid purification, chemical sample analysis, food analysis, and/or forensic analysis. Molecular diagnostics may for example be accomplished with the help of magnetic beads or fluorescent particles that are directly or indirectly attached to target molecules.

### BRIEF DESCRIPTION OF THE DRAWINGS

These and other aspects of the invention will be apparent from and elucidated with reference to the embodiments described hereinafter.

In the drawings:
- Fig. 1: schematically shows a side view of a first biosensor according to the present invention during the mixing of a fluid by magnetically manipulated magnetic particles;
- Fig. 2: shows the biosensor of Figure 1 when the magnetic particles are attracted to a surface;
- Fig. 3: shows a top view onto the magnetic field generator of the biosensor of Figure 1;
- Fig. 4: illustrates consecutive processing steps of a DNA purification procedure;
- Fig. 5: shows exemplary time courses of drive currents that may be applied to the coils in Figures 1-3.;
- Fig. 6: shows a second embodiment of a biosensor in which magnetic subunits are disposed below and above a cartridge.

Like reference numbers or numbers differing by integer multiples of 100 refer in the Figures to identical or similar components.

### DETAILED DESCRIPTION OF EMBODIMENTS

In the field of miniaturized microfluidic devices for analytical chemistry and biotechnology applications, a rapid and efficient mixing is one of the important challenges. In fact, the absence of turbulence at small scale restricts the mixing mechanism to that of molecular diffusion, which is a slow process.

To address the aforementioned issue, the present invention proposes a new microfluidic mixing concept which is based on the manipulation of magnetic particles (beads) by a local alternating magnetic field. Preferably, the magnetic particles form self-assembled structures by magnetic dipole interaction in the presence of a magnetic field. These structures or objects are also called "magneto-rheological structures". Such a structure is usually composed of a very rich and complicated network of magnetic chains, and its size is determined by several competing magnetic forces. Mixing of a medium may then particularly be the result of the chaotic convection generated by a magneto-rheological structures network.

To achieve an efficient (good and fast) mixing of biomolecules with buffers or other media, the present invention particularly proposes to actuate magnetic particles (beads) with the help of electromagnets using (at least) four magnetic subunits. Via this method mixing can be accomplished efficiently and with high yield in (closed) cartridges. This method can also be used for the purification of DNA (or other nucleic acid substances) by using the binding of DNA to magnetic beads.

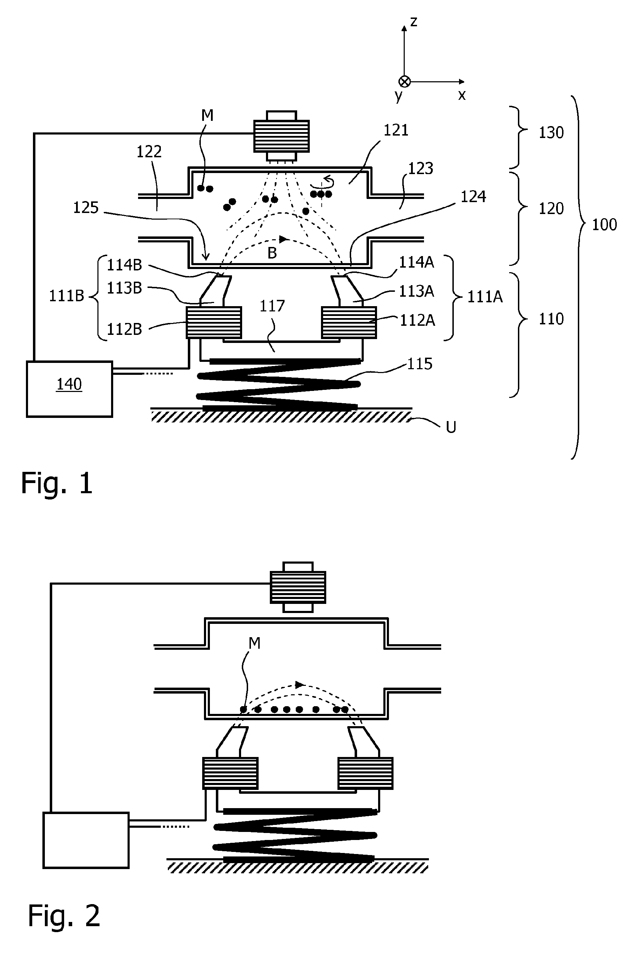

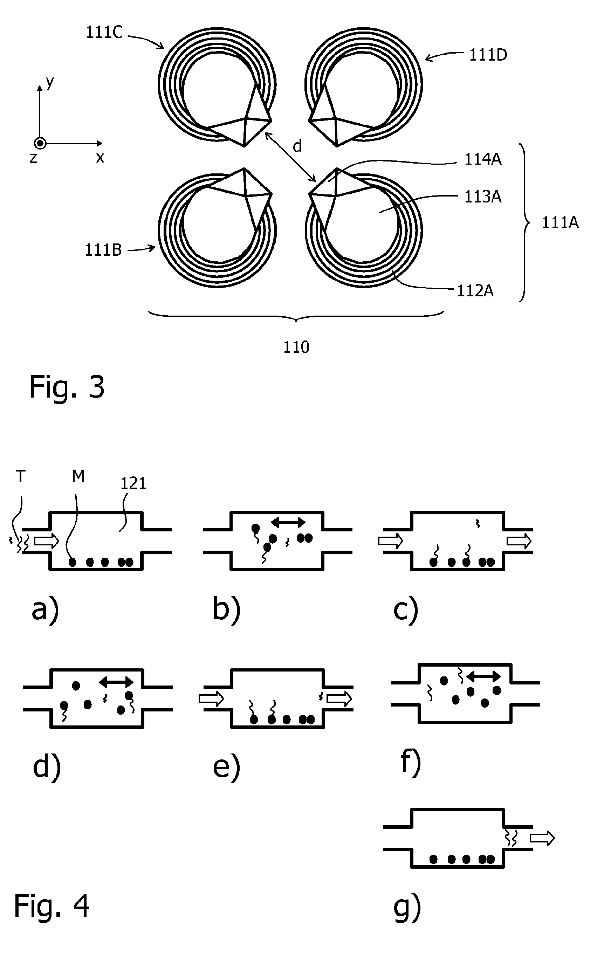

Figures 1-3 show schematically a first embodiment of a processing device 100 according to the above general concepts. The processing device 100 comprises three main components, namely:
1. A cartridge 120 with a processing chamber 121 in which a medium can be provided (typically a fluid, particularly a liquid). The processing chamber 121 is connected to an inlet 122 and an outlet 123 for filling and emptying it, respectively. Moreover, the processing chamber 121 comprises a planar (bottom) wall 124 that has a surface 125 on the side facing the interior of the processing chamber 121. If the processing device 100 is provided with appropriate detection means (e.g. an FTIR detector, not shown), this surface might for example serve as a detection surface.
2. A "multipole magnetic field generator" 110 comprising four magnetic subunits 111A, 111B, 111C, and 111D (in Figures 1 and 2, only the two subunits in the front are visible; all four subunits can be seen in the top view of Figure 3). All four subunits have an identical design, with for example the subunit 111A comprising a core 113A that is surrounded by a coil 112A and that has a top surface 114A. The cores 113A, 113B of the four subunits are parallel to each other (all extending in z-direction). Moreover, the top surfaces 114A-114D are all arranged in the same plane. Accordingly, the four top surfaces can be disposed immediately adjacent to the planar wall 124 of the cartridge 120. The inner distance d between the top surfaces or tips (magnetic air gap) typically ranges between about 1 mm and about 5 cm. The tip width is a critical parameter, since it determines the localization of the generated magnetic field. As there are just four subunits in this embodiment, the magnetic field generator will in the following also be called a "quadrupole".

In an alternative embodiment, the top surfaces 114A-114D might also protrude into the cartridge to surround the processing chamber. In this case, the distance d between the top surfaces of the tips has to be sufficiently large, e.g. about 1.5 cm.
3. An optional additional magnetic field generator 130, for example an electromagnet with a coil and a core, that is disposed above the processing chamber 121. While the Figure shows a single electromagnet, the additional magnetic field generator 130 might also be a multipole (e.g. quadrupole) magnet.

As shown in Figure 1, the cores of all four magnetic subunits 111A-114D may be attached to a common base structure 117. This base structure 117 and/or the cores are preferably made from a ferromagnetic material. More information about possible design features of the single subunits may be found in the US 2011/0221427 A1 and the WO 2010/044006A2, which are incorporated into the present text by reference.

The coils 112A-112D and the additional magnetic field generator 130 are coupled to a control unit 140 by which individual drive currents can be supplied to them. Thus it is possible to control the currents independently of each other and to generate for example a very irregular external magnetic field in the cartridge (which may be favorable for mixing with magnetic particles).

Figure 1 further shows that the whole quadrupole magnetic field generator 110 is carried by a spring 115 that is resilient in z-direction. Accordingly, the quadrupole magnetic field generator 110 and the cartridge 120 can be brought into a perfect contact, irrespective of any manufacturing tolerances, with the top surfaces 114A-114D touching the cartridge 120.

Using a four-subunit magnetic arrangement of electromagnets, mixing of a medium by magnetic particles can be achieved by a quadrupole magnet 110 and optionally an additional top magnet 130 and appropriate actuation of the individual electromagnets or coils 112A-114D, 130. In particular, a varying or oscillating (e.g. rotating) magnetic field B can be generated in-plane.

A key distinguishing feature of the described embodiment is that the quadrupole magnet assembly is below the surface of the cartridge. This ensures that the quadruple magnet works efficiently, while being outside of the cartridge, and that it is positioned in such a way that inserting of the cartridge in the field of the magnet can be done in a simple way.

It should be noted that the actuation of the electromagnets 112A-114D, 130 can also be used the "fix" magnetic particles on the surface 125 for washing applications, when residual liquids are removed/pumped. This is needed in certain steps of the washing and clean-up protocol as described in more detail below.

A particularly advantageous aspect of using electromagnets as subunits is that the shape of the magnetic poles (top surfaces) of the electromagnets does not have to be machined to very high precision. Instead, the desired fields and filed gradients can be generated through appropriate actuation protocols.

Another important factor is the possibility to induce a rotational motion of the magnetic beads by using an alternating magnetic field. This rotational motion offers an additional active contribution to the mixing. Retention and manipulation of magnetic particles in a chamber or channel necessitates a much localized magnetic field. Therefore, the described electromagnetic system allows for the generation and focusing of the magnetic flux. In case of active mixing, the beads are being manipulated magnetically. Fluid in the magneto-rheological structures region (between tips) will be strongly stirred. In this context, one can imagine that the mixing is the result of the chaotic splitting of the fluid by the superparamagnetic beads network. Another important factor is the collective dynamical behavior of the superparamagnetic beads in an alternating magnetic field at low frequencies f (1 Hz < f < 50 Hz). Briefly, this dynamics is the result of the rotation of the magnetic dipoles induced by the changing magnetic field polarity. A time-dependent magnetic field can readily be set-up to induce such motion of the network of the superparamagnetic beads.

A useful application of the described system is the efficient clean-up and removal of short oligo- and DNA fragments, e.g. adapters from a DNA library. Figure 4 illustrates an associated protocol by which a much more efficient clean-up is obtained in a (closed) cartridge, which functions faster and has the advantage of giving a higher yield. A similar protocol could also advantageously be used to improve mixing efficiency, as by the introduction of beads and their rotational moments in a quadrupole field, (rotational) chaotic mixing is introduced.

According to step a) of Figure 4, a processing chamber 121 is filled with a solution containing DNA fragments or targets T. Magnetic particles M that are already present in the chamber are magnetically attracted to the bottom surface of the chamber during this step such that they are not affected by the inflow of the solution.

In step b), the magnetic beads M are released into the medium and actuated for example by a rotating magnetic field in order to start mixing of the fluid in the sample chamber. During this step, the DNA targets T are bound by the magnetic particles M. The magnetic particles typically have a concentration of about 0.2 mg/ml to about 2 mg/ml in the processing chamber.

The aforementioned binding of targets T shall preferably be specific in that only certain target molecules bind while others do not, depending on the properties of the molecules.

In a preferred application, the targets T will for example be comprised of DNA fragments of various lengths, comprising both (desired) long fragments of DNA as well as smaller (unwanted) remainder fragments such as non-ligated adapter fragments, loose oligonucleotides, or too small DNA fragments (not relevant to sequencing as they are below the read length of the following sequencing step) and primer-dimers (used in a possible previously executed PCR step). It is the long DNA fragments which are (should be) captured by the magnetic particles. This can be achieved with the help of magnetic particles that are capable of binding charged molecules electrostatically. This is for example the case for silica beads (in which magnetic nanoparticles are embedded). Only long DNA fragments are sufficiently charged to stick to these magnetic particles (i.e. to the silica).

Additionally or alternatively, magnetic particles can be used that have specific oligonucleotide capture fragments on their surface. In this case, the attraction of DNA or RNA to the magnetic beads takes place by hybridization, in which a nucleic acid fragment binds to its complementary fragment on the surface of a given bead. The capture fragments could be specific for a group of specific nucleic acids, such as poly-dT oligonucleotides for mRNA, or for one specific sequence. By using the proposed active mixing, the capture efficiency of these magnetic beads could be very much improved. This could for example be used in a targeted sequencing effort, i.e. sequencing only the part of the genome of interest for a specific clinical or biological question at hand. The beads used in this process may also be encoded beads as disclosed in WO 2010/097775 A1.

In step c), the magnetic particles M with the bound targets T are attracted to the bottom surface of the processing chamber, which is then emptied by removing residual buffer fluid and small unbound DNA fragments. Moreover, a washing buffer is introduced into the processing chamber while the magnetic particles are still retained at the bottom surface.

In step d), the magnetic particles M with the bound targets T are released again into the processing chamber and actuated for mixing.

In step e), the magnetic particles with the bound targets are attracted to the bottom surface while the processing chamber is emptied from the washing buffer. Moreover, an elution buffer is next introduced into the processing chamber.

In step f), the magnetic particles have been released into the processing chamber and are actuated for mixing the fluid. In this step, the targets T separate from the magnetic particles M.

Experiments indicate that captured target components like DNA can efficiently be released from the magnetic particles even if the latter remain largely in an agglomerated state. This allows to use a processing device with a single magnetic field generator on only one side (e.g. the processing device 100 of Figures 1-3 without the additional magnet 130).

In step g), the magnetic particles are again attracted to the bottom surface, while the medium in the processing chamber comprising the released targets T is removed from said chamber.

It should be noted that the described mixing of a medium by actuating magnetic beads can also be used for many other purposes, for example to lyse cells so that cell components such as DNA and RNA will be released.

An important advantage of the used quadrupole magnet is the ability to manipulate the beads. This is a key for mixing and clean-up steps in the sequencing (and other protocols). To properly up-concentrate and mix a medium, a quadrupole magnet is needed as it allows to circulate beads in the fluid using various driving schemes (e.g. using a block driving scheme or a sinus driving scheme). Experiments show that these driving schemes generate for example oscillating rings of beads, which induce a good mixing of the fluid.

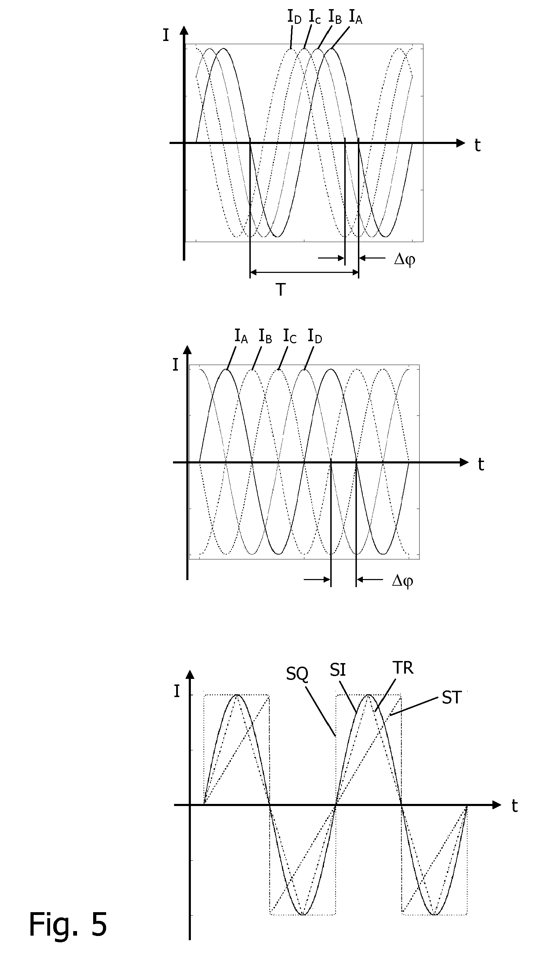

Figure 5 shows the time courses of the driving currents to the four coils 112A-112D for various exemplary driving schemes.

The upper diagram of Figure 5 shows sinusoidal drive currents I_{A}, I_{B}, I_{C}, I_{D} that are applied to the coils 112A, 112B, 112C, 112D, respectively. The four currents have identical amplitudes (e.g. ± 2A) and periods T (e.g. 1 s), but mutual phase shifts Δϕ (of about T/8) between I_{A} and I_{B}, I_{B} and I_{C}, and I_{C} and I_{D}.

The middle diagram of Figure 5 shows the same sinusoidal drive currents I_{A}, I_{B}, I_{C}, I_{D} as the top diagram but with larger mutual phase shifts Δϕ of about T/4.

The bottom diagram of Figure 5 illustrates several basic waveforms that can be used for the drive currents I_{A}, I_{B}, I_{C}, I_{D}, namely:
- a square waveform SQ;
- a sinusoidal waveform SI;
- a triangular waveform TR that is mirror-symmetric to the vertical axis;
- a sawtooth waveform ST (i.e. a triangular waveform with one vertical edge).

As illustrated in the upper two diagrams for the sinusoidal waveform SI, the drive currents for all coils will typically have the same waveform but a mutual phase shift. In general, it is however also possible to use different amplitudes, periods, phase shifts, and/or waveforms for the drive currents of the coils. Moreover, the applied waveforms may also be irregular, i.e. have no periodicity at all.

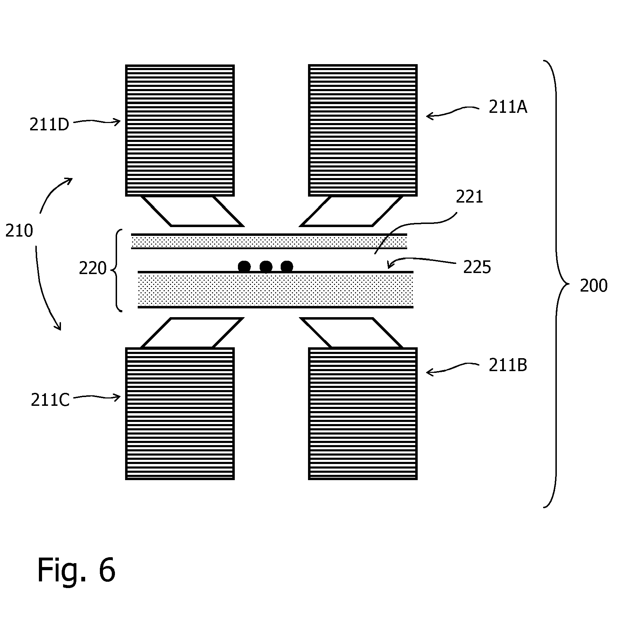

Figure 6 shows a biosensor 200 according to another preferred embodiment of the invention. In the biosensor 200, four subunits 211A, 211B, 211C, 211D (electromagnets) of a multipole magnetic field generator 210 are disposed around a cartridge 220 in order to induce any field orientation that is desired. This enables adjusting fields also in a vertical direction (z-direction).

The described magnetic construction may optionally be combined with heating elements. This may further improve the specificity of the mixing and clean-up step as DNA binding is a function of the temperature T.

In summary, the invention relates to a magnetic field generator, a processing device, and a method for processing a medium in a processing chamber. The processing comprises the addition of magnetic particles to the medium and the mixing of the medium by manipulating said magnetic particles with a time-variable magnetic field, particularly a (partially) oscillating or rotating field. The magnetic field may be generated with a magnetic field generator comprising four subunits, each having a core with a surrounding coil and with a top surface, wherein all top surfaces of said subunits are arranged in the same plane and wherein all cores are substantially parallel to each other. The invention comprises the use of a quadruple magnetic below a cartridge surface to actuate magnetic particles in a (closed) cartridge to effect processing steps such as mixing. Moreover, it relates to a protocol for cleaning up DNA or other nucleic acids such as RNA in a (closed) cartridge using a quadrupole magnet.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method for processing a medium comprising target particles (T), said method comprising the following steps:
a) providing a cartridge (120, 220) having a processing chamber (121, 221);
b) providing a multipole magnetic field generator (110, 210) having at least four subunits (111A, 111B, 111C, 111D, 211A, 211B, 211C, 211D), each subunit having a core (113A, 113B) with a top surface (114A, 114B) and a coil (112A, 112B) surrounding said core (113A, 113B), wherein the top surfaces (114A, 114B) of all subunits are arranged adjacent to the cartridge (120, 220);
c) filling the processing chamber (121, 221) with the medium comprising the target particles (T), wherein the filling is done before, during, and/or after adding magnetic particles (M) to the medium;
d) controlling the multipole magnetic field generator (110, 210) such that a time-variable magnetic field (B) is generated that manipulates the magnetic particles (M) and thus mixes the medium;
e) binding target particles (T) of the medium to the magnetic particles (M);
f) controlling the multipole magnetic field generator (110, 210) such that the magnetic particles (M) are attracted to a surface (125, 225) of the processing chamber (121, 221) and removing the remaining medium from the processing chamber (121, 221).

2. A processing device (100, 200) for processing a medium comprising target particles (T), comprising:
- a cartridge (120, 220) with a processing chamber (121, 221) in which the medium can be provided;
- a multipole magnetic field generator (110, 210) with at least four subunits (111A, 111B, 111C, 111D, 211A, 211B, 211C, 211D), each subunit having a core (113A, 113B) with a top surface (114A, 114B) and a coil (112A, 112B) surrounding said core (113A, 113B), wherein the top surfaces (114A, 114B) of all subunits are arranged adjacent to the cartridge (120, 220);
- a control unit (140) by which individual drive currents can be supplied to the subunits (111A, 111B, 111C, 111D, 211A, 211B, 211C, 211D) of the multipole magnetic field generator (110, 210), comprising drive currents by which
a) magnetic particles (M) are manipulated to mix a medium in the processing chamber (121, 221);
b) magnetic particles (M) are attracted to a surface (125, 225) of the processing chamber (121, 221).

3. The processing device (100, 200) according to claim 2,
**characterized in that** the at least four subunits (111A, 111B, 111C, 111D, 211A, 211B, 211C, 211D) of the multipole magnetic field generator (110, 210) are arranged below the processing chamber (221) and/or surround the processing chamber (221).

4. The processing device (100) according to claim 2,
**characterized in that** an additional magnetic field generator (130) is arranged opposite to the multipole magnetic field generator (130) with respect to the processing chamber (121).

5. The processing device (100, 200) according to claim 2,
**characterized in that** the magnetic particles (M) form magneto-rheological structures while they are manipulated to mix the medium.

6. The processing device (100, 200) according to claim 2,
**characterized in that** the magnetic field (B) comprises at least one oscillating component and/or that it is at least partially rotating.

7. The processing device (100, 200) according to claim 2,
**characterized in that** at least some of the magnetic particles (M) comprise binding sites that specifically bind to target components (T) of the medium and/or that at least some of the magnetic particles (M) are capable of electrostatically binding particles, particularly fragments of nucleic acids.

8. The method according to claim 1,
**characterized in that** a new medium is introduced into the processing chamber (121, 221) after step f).

9. The method according to claim 8,
**characterized in that** target components (T) bound to magnetic particles (M) are released into the new medium.

10. The method according to claim 1,
**characterized in that** the target particles (T) comprise nucleic acids, proteins, polypeptides, lipids, carbohydrates, metabolites, hormones, drugs, pharmaceutical materials, cell fragments, cells, or tissue elements.

11. The processing device (100, 200) according to claim 2,
**characterized in that** the drive currents have a sinusoidal, square, triangle, sawtooth, or irregular time course.

12. A processing device (100, 200) for processing a medium comprising target particles (T), comprising:
- a cartridge (120, 220) with a processing chamber (121, 221) in which the medium can be provided;
- a multipole magnetic field generator (110, 210) with at least four subunits (111A, 111B, 111C, 111D, 211A, 211B, 211C, 211D), each subunit having a core (113A, 113B) with a top surface (114A, 114B) and a coil (112A, 112B) surrounding said core (113A, 113B), wherein the top surfaces (114A, 114B) of all subunits are arranged adjacent to the cartridge (120, 220);
- an additional magnetic field generator (130) that is arranged opposite to the multipole magnetic field generator (130) with respect to the processing chamber (121);
- a control unit (140) by which individual drive currents can be supplied to the subunits (111A, 111B, 111C, 111D, 211A, 211B, 211C, 211D) of the multipole magnetic field generator (110, 210) and/or to the additional magnetic field generator (130).

13. Use of the processing device (100, 200) according to claim 12 for mixing a medium by actuating magnetic particles (M).

14. Use of the processing device (100, 200) according to claim 12 for the separation of target components (T) from a medium.

15. Use of the processing device (100, 200) according to claim 2 for molecular diagnostics, biological sample analysis, nucleic acid processing, chemical sample analysis, food analysis, and/or forensic analysis.
